(19) **Europäisches Patentamt European Patent Office Office européen des brevets**

(11) **EP 4 785 929 A1**

(12) **EUROPEAN PATENT APPLICATION**
published in accordance with Art. 153(4) EPC

(43) Date of publication:
05.08.2026 Bulletin 2026/32

(21) Application number: 24870686.3

(22) Date of filing: 24.09.2024

(51) International Patent Classification (IPC):
*A61K 9/127* (2025.01)   *A61K 47/24* (2006.01)
*A61K 47/28* (2006.01)   *A61K 31/575* (2006.01)
*A61P 3/04* (2006.01)   *A61K 8/14* (2006.01)
*A61K 8/55* (2006.01)   *A61K 8/63* (2006.01)
*A61Q 19/06* (2006.01)

(52) Cooperative Patent Classification (CPC):
A61K 8/14; A61K 8/55; A61K 8/63; A61K 9/127;
A61K 31/575; A61K 47/24; A61K 47/28;
A61P 3/04; A61Q 19/06

(86) International application number:
PCT/CN2024/120596

(87) International publication number:
WO 2025/067142 (03.04.2025 Gazette 2025/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.09.2023 CN 202311266652

(71) Applicants:
• Sichuan Huiyu Pharmaceutical Co., Ltd.
Neijiang, Sichuan 641000 (CN)
• Sichuan Huiyu Yueying Medical Technology Co., Ltd.
Chengdu, Sichuan 610200 (CN)

(72) Inventors:
• ZHANG, Xiangfei
Neijiang, Sichuan 641000 (CN)
• XU, Shanghu
Neijiang, Sichuan 641000 (CN)

• MENG, Guodong
Neijiang, Sichuan 641000 (CN)
• LI, Ruiru
Neijiang, Sichuan 641000 (CN)
• ZHANG, Xingwang
Neijiang, Sichuan 641000 (CN)
• DAI, Tian
Neijiang, Sichuan 641000 (CN)
• YANG, Ya
Neijiang, Sichuan 641000 (CN)
• LI, Yuhuang
Neijiang, Sichuan 641000 (CN)
• LI, Haoyu
Neijiang, Sichuan 641000 (CN)
• DING, Zhao
Neijiang, Sichuan 641000 (CN)

(74) Representative: Delbarba, Andrea
Bugnion S.p.A.
Viale Lancetti, 17
20158 Milano (IT)

(54) **LIPOSOME DRUG DELIVERY SYSTEM AND PREPARATION METHOD THEREFOR, LIPOSOME FORMULATION, USE, AND FAT REDUCTION DRUG OR MEDICAL AESTHETIC PRODUCT COMPRISING LIPOSOME FORMULATION**

(57) A liposome drug delivery system and a preparation method therefor, and a liposomal formulation, a use, and a fat-reducing drug or medical aesthetic product including a liposomal formulation. The liposome drug delivery system includes deoxycholic acid and phospholipid; the phospholipid includes a first phospholipid and optionally includes a second phospholipid; and a mass ratio of the deoxycholic acid to the phospholipid is 1:2.5 to 1:35. The liposomal formulation may further be prepared by using the drug delivery system, and be applied to a fat-reducing drug or medical aesthetic product. The liposome drug delivery system, the liposomal formulation drug or medical aesthetic product has excellent adipocyte selectivity, achieves a good effect of dissolving adipose tissues, has low side effect and high safety, and has good market application prospects.

EP 4 785 929 A1

Fig. 1

**Description**

This application claims the priority to Chinese Patent Application No. 2023112666525 filed on September 27, 2023, the invention of which is hereby incorporated by reference in its entirety.

**Technical Field**

The present invention relates to the field of medical aesthetic products or drugs, and specifically to a liposome drug delivery system and a preparation method therefor, and a liposomal formulation, use, and a fat-reducing drug or medical aesthetic product including a liposomal formulation.

**Background**

Kybella is an injectable solution preparation with deoxycholic acid as its active ingredient, which was approved by the U.S. Food and Drug Administration (FDA) in 2015 for the reduction of moderate to severe submental fat (commonly known as double chin) in adults. The deoxycholic acid achieves its pharmacological effects primarily by causing extensive necrosis of adipocytes by means of lysing cell membranes. However, due to the lack of strong cell-killing specificity of the deoxycholic acid, it damages not only adipocytes, but also other normal non-adipocytes, causing an inflammatory reaction at the injection site. Furthermore, the Kybella itself has a slightly alkaline pH, which significantly increases the incidence of side effects such as severe pain, nodules, swelling, nerve numbness, etc. after injection.

Another category of injectable drugs for localized fat reduction includes representatives such as Essentiale developed by A. Nattermann GmbH in Germany in the 1950s for treating liver diseases, Lipostabil used for preventing and treating fat embolism, and Lipobean approved in South Korea as an adjunctive medication for hepatic coma caused by liver cirrhosis. Such injectable drugs primarily consist of a mixture of polyene phosphatidylcholine and the solubilizing agent deoxycholic acid. During clinical application, it has been found to reduce local fat in patients, leading to its off-label use for localized lipolysis. However, research feedback indicates that the active fat-reducing ingredient in such injections is actually the deoxycholic acid. Its lipolysis effect and side effects remain highly controversial, and clinically it may also cause adverse reactions such as pain, erythema, swelling, induration, etc. The patent CN102970993A discloses in the related art that the disadvantages of such preparation for subcutaneous lipolysis primarily involve adverse reactions such as swelling, hematoma (bruising), pain and discomfort in treatment regions, including a burning sensation and itching at the injection site after treatment, as well as cellular necrosis.

Liposomes may serve as carriers for many drugs. Targeted liposomes may typically reduce drug toxicity. Liposome targeting generally includes two forms: passive targeting and active targeting. Passively-targeted liposomes lack active groups, use the physiological characteristics and differences of human organs to achieve selective enrichment in organs or lesions, and are commonly applied in the field of tumors. Active targeting is difficult to achieve and often requires appropriate modification of liposomes, including antibodies, oligosaccharides, polypeptides, proteins, and vitamins. Research focuses on antibodies, primarily leveraging the high affinity and specificity of monoclonal antibodies to achieve targeted effects.

However, it is not easy to truly realize the therapeutic advantages of liposomes. The following three requirements must be met: 1) the drug can be encapsulated within the liposome with high encapsulation efficiency and sufficient drug loading; 2) the liposome remains stable in vitro; and 3) the drug has good targeting properties. Based on the current liposome technology, developing liposomal formulations that meet all quality requirements while simultaneously achieving targeting properties of liposomal drugs has become a key focus and challenge in research. How to effectively control liposomal drugs to target specific cells is a technical problem that needs to be solved during the development of liposomal drugs.

The industry has previously attempted to research liposomal drugs for localized fat reduction. The Chinese Patent CN1870977A discloses a liposome system for deoxycholic acid, including high-purity soybean phosphatidylcholine and deoxycholic acid. The liposomes have a diameter ranging from 30 nm to 100 nm and have therapeutic efficacy against lipoma and cellulitis. It also discloses that during the treatment process, patients experienced adverse reactions, including mild prickling sensations, tenderness and sensitivity, as well as moderate erythema.

For deoxycholic acid molecules, there are no published reports on liposomes with adipocyte-targeting capabilities. How to target adipocytes by using the deoxycholic acid liposomes is a major challenge, which is also a key point for improving efficacy and reducing toxicity.

**Summary**

In view of the disadvantages in the related art, the present invention is intended to provide a deoxycholic acid liposome with better Encapsulation Efficiency (EE), stability, and adipocyte targeting, thereby achieving a lipolysis effect of improving efficacy and reducing toxicity. As above, since this product is a localized lipolysis drug, it needs to be released at an injection site to ensure therapeutic efficacy at the required site, otherwise it cannot achieve treatment expectations. Additionally, the product needs to actively target adipocytes while minimizing cytotoxic effects on non-adipocytes, thereby reducing toxicity.

In the present invention, extensive research has revealed that a deoxycholic acid liposome prepared in Chinese Patent CN1870977A lacks adipocyte-targeting specificity, which not only kills adipocytes but also has potent cytotoxic effects on non-adipocytes, resulting in significant adverse reactions. The deoxycholic acid liposome provided in the present invention can still show an excellent selective killing effect on adipocytes in adipocyte and vascular endothelial cell cytotoxicity tests without modifications such as antibodies, vitamins, polypeptides, and sugars.

The deoxycholic acid liposome drug composition provided in the present invention exhibits excellent adipocyte selectivity, high safety, high encapsulation efficiency, good stability, and excellent quality.

In a first aspect, the present invention provides a liposome drug delivery system, including deoxycholic acid and phospholipid. The phospholipid includes a first phospholipid and optionally includes a second phospholipid; a mass ratio of the deoxycholic acid to the phospholipid is 1:2.5 - 1:35; preferably, the mass ratio of the deoxycholic acid to the phospholipid is 1:3.4-1:30.4; and more preferably, the mass ratio of the deoxycholic acid to the phospholipid is 1:4.4-1:30.4.

In some embodiments of the present invention, the mass ratio of the deoxycholic acid to the phospholipid is 1:4-1:7, preferably 1:4.3-1:6.5, and more preferably 1:5-1:6.

In the liposome drug delivery system provided in the present invention, the phospholipid includes main phospholipid (also referred to as the first phospholipid) for maintaining phospholipid bilayer, and optionally includes auxiliary phospholipid (also referred to as the second phospholipid) for further modifying surface characteristics of a liposome.

In the liposome drug delivery system provided in the present invention, the first phospholipid is selected from one or more of egg yolk lecithin (PC), soy lecithin (SPC), hydrogenated soy lecithin (HSPC), dioleoylphosphatidylcholine (DOPC), dierucoylphosphatidylcholine (DEPC), distearylphosphatidylcholine (DSPC), 1-palmitoyl-2-oleoyl-phosphatidylcholine (POPC), dimyristoylphosphatidylcholine (DMPC), dipalmitoylphosphatidylcholine (DPPC), distearoylphosphatidylethanolamine (DSPE), and dioleylphosphatidylethanolamine (DOPE).

In some embodiments, in the liposome drug delivery system provided in the present invention, the first phospholipid is selected from soy lecithin (SPC), hydrogenated soy lecithin (HSPC), distearylphosphatidylcholine (DSPC), dimyristoylphosphatidylcholine (DMPC), dipalmitoylphosphatidylcholine (DPPC), dioleoylphosphatidylcholine (DOPC), dierucoylphosphatidylcholine (DEPC), and 1-palmitoyl-2-oleoyl-phosphatidylcholine (POPC), preferably soy lecithin (SPC), dipalmitoylphosphatidylcholine (DPPC), dioleoylphosphatidylcholine (DOPC), dierucoylphosphatidylcholine (DEPC), or 1-palmitoyl-2-oleoyl-phosphatidylcholine (POPC), more preferably dioleoylphosphatidylcholine (DOPC), dierucoylphosphatidylcholine (DEPC), or 1-palmitoyl-2-oleoyl-phosphatidylcholine (POPC), such as dioleoylphosphatidylcholine (DOPC).

In the liposome drug delivery system provided in the present invention, the second phospholipid is selected from one or more of 1,2-distearoyl-sn-glycero-3-phosphocholine (DSPG), dioleoylphosphatidylserine (DOPS), distearoylphosphatidylserine (DSPS), (2-dioleoyl hydroxypropyl)trimethylammonium chloride (DOTAP), 1,2-dioleyloxy-3-dimethylamino-propane (DODMA), distearoylphosphatidylethanolamine-polyethylene glycol 2000 (DSPE-PEG2000), dioleoylphosphatidylglycerol (DOPG), and egg yolk phosphatidylglycerol (EPG), preferably distearoylphosphatidylethanolamine-polyethylene glycol 2000 (DSPE-PEG2000), 1,2-distearoyl-sn-glycero-3-phosphocholine (DSPG), dioleoylphosphatidylglycerol (DOPG), or 1,2-dioleyloxy-3-dimethylamino-propane (DODMA), preferably distearoylphosphatidylethanolamine-polyethylene glycol 2000 (DSPE-PEG2000) or 1,2-distearoyl-sn-glycero-3-phosphocholine (DSPG), more preferably distearoylphosphatidylethanolamine-polyethylene glycol 2000 (DSPE-PEG2000).

In the liposome drug delivery system provided in the present invention, the mass ratio of the deoxycholic acid to the phospholipid is 1:3.4, 1:3.6, 1:4.4, 1:5, 1:5.4, 1:5.5, 1:6, 1:6.4, or 1:30.4.

In the liposome drug delivery system provided in the present invention, the mass ratio of the deoxycholic acid to the first phospholipid is 1:3-1:32, preferably 1:4-1:30, more preferably 1:4-1:6, such as 1:4, 1:5, or 1:6.

In the liposome drug delivery system provided in the present invention, the mass ratio of the deoxycholic acid, the first phospholipid, and the second phospholipid is 1:(3-32):(0-3).

In some embodiments, in the liposome drug delivery system provided in the present invention, the mass ratio of the deoxycholic acid, the first phospholipid, and the second phospholipid is 1:(4-30):(0.2-0.6); and preferably, the mass ratio of the deoxycholic acid, the first phospholipid, and the second phospholipid is 1:(4-30):(0.4-0.5).

In some embodiments, in the liposome drug delivery system provided in the present invention, the mass ratio of the deoxycholic acid, the first phospholipid, and the second phospholipid is 1:(4-10):(0-0.6), preferably 1:(4-6):(0.2-0.6), and more preferably 1:(4-6):(0.4-0.5).

In some embodiments, in the liposome drug delivery system provided in the present invention, the mass ratio of the deoxycholic acid, the first phospholipid, and the second phospholipid is 1:3:0.4, 1:4:0.4, 1:5:0.4, 1:5:0.5, 1:6:0.4, or 1:30:0.4; and preferably, the mass ratio of the deoxycholic acid, the first phospholipid, and the second phospholipid is 1:4:0.4, 1:5:0.4, 1:5:0.5, 1:6:0.4, or 1:30:0.4.

In some embodiments, in the liposome drug delivery system provided in the present invention, the mass ratio of the first phospholipid to the second phospholipid is (3-32):(0-3), preferably, the mass ratio of the first phospholipid to the second phospholipid is (4-30):(0.2-0.6), more preferably 3:0.4, 4:0.4, 5:0.4, 5:0.5, 6:0.4, or 30:0.4.

In the liposome drug delivery system provided in the present invention, the ratio of drug to lipid is 1:(3-32), preferably 1:(4-30.9), such as 1:5.9, 1:3.9, 1:4.0, 1:4.9, 1:6.9, 1:5.5, 1:6, or 1:6.1.

The liposome drug delivery system provided in the present invention further optionally includes cholesterol and/or an antioxidant. Preferably, the antioxidant is selected from vitamin E and/or vitamin E acetate.

The liposome drug delivery system provided in the present invention further includes an aqueous phase. The aqueous phase is selected from one or more of a disodium succinate buffer solution, a histidine solution, normal saline, a sucrose solution, and water for injection.

In some embodiments, in the liposome drug delivery system provided in the present invention, the aqueous phase is selected from one or more of a 0.02-0.04 M disodium succinate buffer solution containing 0.5% NaCl, a 0.05 M disodium succinate buffer solution, a 0.025 M histidine solution, normal saline, and an 8% sucrose solution. The 8% sucrose solution refers to a sucrose aqueous solution with a mass concentration of 8%.

In some embodiments, in the liposome drug delivery system provided in the present invention, the aqueous phase is the 0.02-0.04 M disodium succinate buffer solution containing 0.5% NaCl.

Preferably, the pH value of the 0.02-0.04 M disodium succinate buffer solution containing 0.5% NaCl is 5.0-7.0; and more preferably, the pH value is 6-6.5.

In the liposome drug delivery system provided in the present invention, each unit of the preparation is prepared from the following components: 1-5 parts of the deoxycholic acid, 15-30 parts of the first phospholipid, 0-2 parts of the second phospholipid, and 100-5000 parts of the aqueous phase, preferably 500-5000 parts of the aqueous phase.

In some embodiments, in the liposome drug delivery system provided in the present invention, each unit of the preparation is prepared from the following components: 1-5 parts of the deoxycholic acid, 15-30 parts of the first phospholipid, 0-2 parts of the second phospholipid, 500-5000 parts of the aqueous phase, 0-2.5 parts of cholesterol, and 0-0.625 parts of vitamin E acetate.

In some embodiments, in the liposome drug delivery system provided in the present invention, each unit of the preparation is prepared from the following components: 5 parts of the deoxycholic acid, 15-30 parts of the first phospholipid, 0-2 parts of the second phospholipid, 500 parts of the aqueous phase, 0-2.5 parts of cholesterol, and 0-0625 parts of vitamin E acetate.

In some embodiments, in the liposome drug delivery system provided in the present invention, the first phospholipid is selected from one or more of soy lecithin (SPC), dipalmitoylphosphatidylcholine (DPPC), dioleoylphosphatidylcholine (DOPC), dierucoylphosphatidylcholine (DEPC), and 1-palmitoyl-2-oleoyl-phosphatidylcholine (POPC); preferably, the first phospholipid is selected from one or more of dioleoylphosphatidylcholine (DOPC), dierucoylphosphatidylcholine (DEPC), and 1-palmitoyl-2-oleoyl-phosphatidylcholine (POPC), more preferably dioleoylphosphatidylcholine (DOPC).

In some embodiments, in the liposome drug delivery system provided in the present invention, the second phospholipid is selected from one or more of 1,2-distearoyl-sn-glycero-3-phosphocholine (DSPG), dioleoylphosphatidylserine (DOPS), distearoylphosphatidylserine (DSPS), (2-dioleoyl hydroxypropyl)trimethylammonium chloride (DOTAP), 1,2-dioleyloxy-3-dimethylamino-propane (DODMA), distearoylphosphatidylethanolamine-polyethylene glycol 2000 (DSPE-PEG2000), dioleoylphosphatidylglycerol (DOPG), and egg yolk phosphatidylglycerol (EPG); preferably, the second phospholipid is selected from one or more of distearoylphosphatidylethanolamine-polyethylene glycol 2000 (DSPE-PEG2000) and 1,2-distearoyl-sn-glycero-3-phosphocholine (DSPG), more preferably distearoylphosphatidylethanolamine-polyethylene glycol 2000 (DSPE-PEG2000).

In some embodiments, in the liposome drug delivery system provided in the present invention, the aqueous phase is selected from one or more of the 0.02-0.04 M disodium succinate buffer solution containing 0.5% NaCl, the 0.05 M disodium succinate buffer solution, the 0.025 M histidine solution, the normal saline, and the 8% sucrose solution; preferably, the aqueous phase is selected from one or more of the 0.02-0.04 M disodium succinate buffer solution containing 0.5% NaCl, the 0.025 M histidine solution, the normal saline, and the 8% sucrose solution; more preferably, the aqueous phase is the 0.02-0.04 M disodium succinate buffer solution containing 0.5% NaCl.

More preferably, the pH value of the 0.02-0.04 M disodium succinate buffer solution containing 0.5% NaCl is 5.0-7.0; and preferably, the pH value is 6-6.5.

In some embodiments, in the liposome drug delivery system provided in the present invention, the first phospholipid and the second phospholipid meet any one of the following combinations:

(1) soy lecithin (SPC) and distearoylphosphatidylethanolamine-polyethylene glycol 2000 (DSPE-PEG2000);

(2) hydrogenated soy lecithin (HSPC) and distearoylphosphatidylethanolamine-polyethylene glycol 2000 (DSPE-PEG2000);

(3) distearylphosphatidylcholine (DSPC), and distearoylphosphatidylethanolamine-polyethylene glycol 2000 (DSPE-PEG2000);

(4) dierucoylphosphatidylcholine (DEPC) and distearoylphosphatidylethanolamine-polyethylene glycol 2000 (DSPE-PEG2000);

(5) 1-palmitoyl-2-oleoyl-phosphatidylcholine (POPC) and distearoylphosphatidylethanolamine-polyethylene glycol 2000 (DSPE-PEG2000);

(6) dimyristoylphosphatidylcholine (DMPC) and distearoylphosphatidylethanolamine-polyethylene glycol 2000 (DSPE-PEG2000);

(7) dipalmitoylphosphatidylcholine (DPPC) and distearoylphosphatidylethanolamine-polyethylene glycol 2000 (DSPE-PEG2000);

(8) dioleoylphosphatidylcholine (DOPC) and distearoylphosphatidylethanolamine-polyethylene glycol 2000 (DSPE-PEG2000);

(9) dioleoylphosphatidylcholine (DOPC) and 1,2-distearoyl-sn-glycero-3-phosphocholine (DSPG);

(10) dioleoylphosphatidylcholine (DOPC) and dioleoylphosphatidylglycerol (DOPG); and

(11) dioleoylphosphatidylcholine (DOPC) and 1,2-dioleyloxy-3-dimethylamino-propane (DODMA).

In some embodiments, in the liposome drug delivery system provided in the present invention, the first phospholipid is dioleoylphosphatidylcholine (DOPC), the second phospholipid is distearoylphosphatidylethanolamine-polyethylene glycol 2000 (DSPE-PEG2000), and the mass ratio of the first phospholipid to the second phospholipid is (4-6):(0.4-0.6); the mass ratio of the first phospholipid to the second phospholipid is preferably (4-6):(0.4-0.5), such as 5:0.4 or 5:0.5.

Alternatively, the first phospholipid is dierucoylphosphatidylcholine (DEPC), the second phospholipid is distearoylphosphatidylethanolamine-polyethylene glycol 2000 (DSPE-PEG2000), and the mass ratio of the first phospholipid to the second phospholipid is (4-6):(0.4-0.6); the mass ratio of the first phospholipid to the second phospholipid is preferably (4-6):(0.4-0.5), such as 5:0.4 or 5:0.5.

Alternatively, the first phospholipid is 1-palmitoyl-2-oleoyl-phosphatidylcholine (POPC), the second phospholipid is distearoylphosphatidylethanolamine-polyethylene glycol 2000 (DSPE-PEG2000), and the mass ratio of the first phospholipid to the second phospholipid is (4-6):(0.4-0.6); the mass ratio of the first phospholipid to the second phospholipid is preferably (4-6):(0.4-0.5), such as 5:0.4 or 5:0.5.

In a second aspect, the present invention provides a liposomal formulation, including the above liposome drug delivery system. The preparation is an injection or a lyophilized preparation.

In some embodiments, for the liposomal formulation provided in the present invention, when the preparation is the lyophilized preparation, the preparation further optionally includes a lyophilized excipient; and the lyophilized excipient is selected from one or more of sucrose, lactose, trehalose, and mannitol, preferably sucrose.

In a third aspect, the present invention further provides a method for preparing the foregoing liposome drug delivery system.

The method for preparing the foregoing liposome drug delivery system provided in the present invention may be a conventional method in the art, which is preferably selected from an injection method, a reverse phase evaporation method, a thin-film dispersion method, and an active drug loading method.

In some embodiments, the preparation method provided in the present invention preferably includes the following steps.

At I, deoxycholic acid, the first phospholipid, the second phospholipid, cholesterol, and an antioxidant (e.g., vitamin E acetate) are taken and dissolved according to a ratio in an organic solvent to prepare an organic phase.

At II, the organic solvent in the organic phase obtained in step I is removed, and an aqueous phase is added to the organic phase for hydration, so as to obtain a crude suspension.

At III, the crude suspension obtained in step II is homogenized, so as to obtain a suspension.

At IV, the suspension obtained in step III is filtered through either a 0.45 $\mu$m filter membrane or a (1.0+0.65) $\mu$m

double-layer filter membrane, and is subsequently filtered through a 0.22 μm microporous filter membrane, so as to obtain a liposome.

In some embodiments, the total solid content of the organic phase in step I is 20% (w/v).

In some embodiments, the organic solvent in step I is selected from one or more of chloroform, anhydrous ethanol, and anhydrous methanol, preferably a mixed solution of chloroform and anhydrous methanol. In the mixed solution of chloroform and anhydrous methanol, a volume ratio of the chloroform to the anhydrous methanol is preferably 1:1-30:1, such as 10:1.

In some embodiments, the amount of the organic solvent in step I is typically sufficient to achieve the total solid content of 20% (w/v) in the formed organic phase.

In some embodiments, the aqueous phase in step II is selected from one or more of a 0.02-0.04 M disodium succinate buffer solution containing 0.5% NaCl, a 0.05 M disodium succinate buffer solution, a 0.025 M histidine solution, normal saline, and an 8% sucrose solution; more preferably, the aqueous phase is the 0.02-0.04 M disodium succinate buffer solution containing 0.5% NaCl; more preferably, the pH value of the 0.02-0.04 M disodium succinate buffer solution containing 0.5% NaCl is 5.0-7.0 (such as 6.0-6.5).

In some embodiments, the amount of aqueous phase in step II is determined based on the amount of the deoxycholic acid. The amount of the aqueous phase preferably causes the mass concentration of the deoxycholic acid in the liposome solution to be 5-20 mg/mL, more preferably 10 mg/mL.

In some embodiments, the amount of aqueous phase in step II is preferably 80%-95% of the total volume of the liposome, such as 90%.

In some embodiments, the method for removing the organic solvent in step II is reduced-pressure distillation; the reduced-pressure distillation is preferably performed at 45-60 °C. The purpose of reduced-pressure distillation is to remove the organic solvent to form a lipid film.

In some embodiments, the temperature for hydration in step II is 20-60 °C, preferably 50-60 °C or 20-25 °C.

In some embodiments, the time for hydration in step II is 60-120 min.

In some embodiments, the homogenizing condition in step III is to perform homogenizing for 3-5 times at 8000-12000 psi.

In some embodiments, in step IV, the suspension obtained in step III is filtered through the 0.45 μm filter membrane or large particles are filtered through the double-layer filter membrane of (1.0+0.65) μm, and then filtration sterilization is performed through the 0.22 μm microporous filter membrane, so as to obtain the liposome.

In another aspect, the present invention further provides a method for preparing a liposomal formulation. The liposomal formulation is a lyophilized preparation. The preparation method includes the method for preparing a liposome drug delivery system. After homogenizing in step III and before filtration using a filter membrane in step IV, the suspension is mixed with the lyophilized excipient, and then an aqueous phase (e.g., a 0.025 M disodium succinate buffer solution containing 0.5% NaCl at pH 6-6.5) is added; then filtration is performed through a 0.45 μm filter membrane, then filtration is performed through a 0.22 μm microporous filter membrane, so as to obtain a liposome, and the liposome is lyophilized.

In some embodiments, after the lyophilized excipient is completely dissolved, the aqueous phase is added.

In some embodiments, after mixing with the lyophilized excipient, the adding of the aqueous phase preferably causes a weight percentage content of the lyophilized excipient in the solution to reach 5%-8%, such as 6%.

In some embodiments, in the liposome drug delivery system or liposomal formulation provided in the present invention, an average particle size of the liposome is 50-300 nm, preferably 50-100 nm; more preferably, the average particle size of the liposome is 60-95 nm, and most preferably 60-91.5 nm.

In the liposome drug delivery system or liposomal formulation provided in the present invention, the encapsulation efficiency of the liposome may reach above 75%, preferably above 85%, more preferably above 90%, and most preferably above 95%.

In the liposome drug delivery system or liposomal formulation provided in the present invention, a Polydispersity Index (PDI) of the liposome may be 0.1-0.4, preferably 0.1-0.35, more preferably 0.1-0.25, and most preferably 0.1-0.2.

In the liposome drug delivery system or liposomal formulation provided in the present invention, a Zeta potential of the liposome may reach -65 mV to -5 mV, preferably -45mV to -20 mV, and more preferably -45 mV to -35 mV.

In a fourth aspect, the present invention provides use of the liposome drug delivery system or the liposomal formulation in preparation of a fat-reducing drug or a medical aesthetic product. Preferably, the fat-reducing drug or medical aesthetic product are a localized fat-reducing drug or a localized medical aesthetic product.

In some embodiments, in the use provided in the present invention, the fat-reducing drugs or medical aesthetic products crack cell membranes of adipocytes and/or promote apoptosis of the adipocytes. Preferably, the fat-reducing drugs or medical aesthetic products are taken up by the adipocytes.

In a fifth aspect, the present invention provides a fat-reducing drug or medical aesthetic product, including the above liposome drug delivery system or the liposomal formulation, as well as an acceptable excipient or auxiliary component in the fat-reducing drug or medical aesthetic product.

In some embodiments, in the fat-reducing drug or medical aesthetic product provided in the present invention, the excipient or auxiliary component is selected from one or more of VC, benzoic acid, and lidocaine.

In some embodiments, in the fat-reducing drug or medical aesthetic product provided in the present invention, the fat-reducing drug or medical aesthetic product is a liposomal formulation for local injection into adipose tissue.

The deoxycholic acid liposome drug delivery system and the liposomal formulation or medical aesthetic product including the same provided in the present invention have good fat-reducing effects. By optimizing the composition of the phospholipid in the liposome, the selectivity of the adipocytes can be improved, an effect of dissolving adipose tissue is achieved, and the liposome drug delivery system and the fat-reducing drug or medical aesthetic product including the same have low side effects and high safety, and have good market application prospects.

The liposomal formulation for local injection into adipose tissue provided in the present invention can efficiently dissolve the adipose tissue, reduce fat accumulation, reduce the killing of other non-adipocytes (e.g., vascular cells, etc.), and its therapeutic effect is far superior to that of commercially available lipolysis needles, while its adverse reactions are far fewer than those of the latter.

**Brief Description of the Drawings**

Fig. 1 shows the morphology of a deoxycholic acid liposome under an electron microscope.

Fig. 2 shows comparison of killing capability against adipocytes and HUVEC (Human Umbilical Vein Endothelial Cells).

Fig. 3 shows comparison of in-vivo lipolysis effects and adverse reactions to tail adipose tissue of rats.

**Detailed Description of the Embodiments**

Abbreviation meanings:

PC: egg yolk lecithin

SPC: soy lecithin

DOPC: dioleoylphosphatidylcholine, also known as dioleoyl lecithin

DSPC: distearylphosphatidylcholine

HSPC: hydrogenated soy lecithin

DEPC: dierucoylphosphatidylcholine

DMPC: dimyristoylphosphatidylcholine

DPPC: dipalmitoylphosphatidylcholine

POPC: 1-palmitoyl-2-oleoyl-phosphatidylcholine

DSPG: distearoylphosphatidylglycerol

DOPG: dioleoylphosphatidylglycerol

DSPE-PEG2000: distearoylphosphatidylethanolamine-polyethylene glycol 2000

DPPC: dipalmitoylphosphatidylcholine

DSPE: distearoylphosphatidylethanolamine

DOPE: dioleylphosphatidylethanolamine

DOPS: dioleoylphosphatidylserine

DSPS: distearoylphosphatidylserine

DOTAP: (2-dioleoyl hydroxypropyl)trimethylammonium chloride

DODMA: 1,2-dioleyloxy-3-dimethylamino-propane

EPG: egg yolk phosphatidylglycerol

PS: phosphatidylserine

Definitions and terms:
Encapsulation Efficiency (EE) refers to a ratio of a drug encapsulated in a liposome to a dosage during drug loading.

$$\text{Calculation formula: EE} = \text{W inside of liposome/W total of input} \times 100\%$$

Drug loading (DL) refers to a ratio of the mass of the drug encapsulated in the liposome to the total mass of the liposomal formulation.

$$\text{Calculation formula: DL} = \text{W inside of liposome/W total} \times 100\%$$

Drug:Lipid (D:L) refers to a ratio of the mass of the drug to the total mass of lipid components (phospholipid and cholesterol).

In the present invention, when it is described as "... solution," unless otherwise specified, it refers to an aqueous solution of a particular substance. For example: "disodium succinate buffer solution, histidine solution, and sucrose solution" respectively refer to an aqueous solution with the disodium succinate as the solute, an aqueous solution with histidine as the solute, and an aqueous solution with sucrose as the solute.

In the present invention, the phrase "optionally including..." refers to substances that may or may not be included.

Aqueous phase: referring to a water-based solution added during a hydration step, or a water-based solution used in liposomal formulation that differs from an organic phase.

Solid content: referring to a mass-to-volume ratio of a solid raw material to a solvent in a solution or mixture, in g/mL. For example, in Example 2 of the present invention, "weighing...to prepare an organic phase with a total solid content of 20% (w/v)" refers to a mass volume percentage (w/v) of the total mass of deoxycholic acid, soy lecithin (SPC), distearoylphosphatidylethanolamine-polyethylene glycol 2000 (DSPE-PEG2000), cholesterol, and vitamin E acetate in the organic phase is 20% (g/mL) in anhydrous ethanol.

A "0.02-0.04 M disodium succinate buffer solution containing 0.5% NaCl" in the present invention refers to an aqueous solution containing 0.02-0.04 mol/L disodium succinate, to which NaCl is added such that its mass percentage in the solution is 0.5%. The solution may be further adjusted to a pH of 5-7 using a pH regulator. The pH regulator that may be used includes, but is not limited to, a 1M sodium hydroxide solution and a 1M hydrochloric acid solution.

The normal saline in the present invention indicates a sodium chloride solution with an osmotic pressure essentially equivalent to that of animal or human plasma, such as a sodium chloride aqueous solution with a mass concentration of 0.9%.

The "liposome drug delivery system" or "liposome" in the present invention refers to a deoxycholic acid liposome solution or suspension prepared through homogenization and filtration steps, which may further be prepared into injections or lyophilized preparations, etc.

It is apparent that, based on the above content of the present invention, various modifications, substitutions, or alterations may be made without departing from the fundamental technical concept of the present invention using ordinary technical knowledge and conventional means in the art.

The above content of the present invention is further described in detail below by the following specific implementations in the forms of examples. However, it should not be understood that the scope of the above subject of the present invention is limited to the following examples. All technologies implemented based on the content of the present invention fall in the scope of the present invention.

**Example 1 (Comparative Example)**

According to Example 1 of Patent CN1870977A, prepared a deoxycholic acid liposome by using soy lecithin (SPC) as a membrane material.

12.5 g of high-purity soy lecithin (SPC) (with a phosphatidylcholine content greater than 95%) and 6.325 g of deoxycholic acid were dissolved in 50 mL ethanol. The obtained solution was then evaporated under reduced pressure, and the residues were dispersed in 250 mL water. The mixture was homogenized to achieve an average particle size of the liposome of 30 -100 nm. The obtained liposome system was filtered through a 0.2 $\mu$m filter under sterile conditions, and encapsulation efficiency, the particle size, PDI, and Zeta potential were measured. Results are shown in Table 1.

**Example 2**

A deoxycholic acid liposome was prepared (the first phospholipid: SPC), with a mass ratio of deoxycholic acid, the first phospholipid, and the second phospholipid of 1:5:0.4.

5 g of the deoxycholic acid, 25 g of soy lecithin (SPC), 2 g of distearoylphosphatidylethanolamine-polyethylene glycol 2000 (DSPE-PEG2000), 2.5 g of cholesterol and 0.625 g of vitamin E acetate were weighed and mixed, the mixture was dissolved in anhydrous ethanol to prepare an organic phase with a total solid content of 20%(w/v), and an organic solvent was removed by vacuum evaporation at 45-60 °C, so as to obtain a lipid film. Then, 450 mL of normal saline (0.9% NaCl) was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the normal saline (0.9%NaCl), the obtained sample was first filtered through a 0.45 $\mu$m filter membrane to remove large particles, then sterilized by filtration through a 0.22 $\mu$m microporous filter membrane, so as to obtain the liposome, and encapsulation efficiency, particle size, PDI, and Zeta potential were measured. Results are shown in Table 1.

**Example 3**

A deoxycholic acid liposome was prepared (the first phospholipid: HSPC), with a mass ratio of deoxycholic acid, the first phospholipid, and the second phospholipid of 1:5:0.4.

5 g of the deoxycholic acid, 25 g of hydrogenated soy lecithin (HSPC), 2 g of distearoylphosphatidylethanolamine-polyethylene glycol 2000 (DSPE-PEG2000), 2.5 g of cholesterol and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration, the hydration temperature was 50-60 °C, the hydration time was 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a 0.45 $\mu$m filter membrane to remove large particles, then sterilized by filtration through a 0.22 $\mu$m microporous filter membrane, so as to obtain the liposome, and encapsulation efficiency, particle size, PDI, and Zeta potential were measured. Results are shown in Table 1.

**Example 4**

A deoxycholic acid liposome was prepared (the first phospholipid: DSPC), with a mass ratio of deoxycholic acid, the first phospholipid, and the second phospholipid of 1:5:0.4.

5 g of the deoxycholic acid, 25 g of distearylphosphatidylcholine (DSPC), 2 g of distearoylphosphatidylethanolamine-polyethylene glycol 2000 (DSPE-PEG2000), 2.5 g of cholesterol and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration, the hydration temperature was 50-60 °C, the hydration time was 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a 0.45 $\mu$m filter membrane to remove large particles, then sterilized by filtration through a 0.22 $\mu$m microporous filter membrane, so as to obtain the liposome, and encapsulation efficiency, particle size, PDI, and Zeta potential were measured. Results are shown in Table 1.

**Example 5**

A deoxycholic acid liposome was prepared (the first phospholipid: DEPC), with a mass ratio of deoxycholic acid, the first phospholipid, and the second phospholipid of 1:5:0.4.

5 g of the deoxycholic acid, 25 g of dierucoylphosphatidylcholine (DEPC), 2 g of distearoylphosphatidylethanolamine-polyethylene glycol 2000 (DSPE-PEG2000), 2.5 g of cholesterol and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a 0.45 $\mu$m filter membrane to remove large particles, then sterilized by filtration through a 0.22 $\mu$m microporous filter membrane, so as to obtain the liposome, and encapsulation efficiency, particle size, PDI, and Zeta potential were measured. Results are shown in Table 1.

**Example 6**

A deoxycholic acid liposome was prepared (the first phospholipid: POPC), with a mass ratio of deoxycholic acid, the first phospholipid, and the second phospholipid of 1:5:0.4.

5 g of deoxycholic acid, 25 g of 1-palmitoyl-2-oleoyl-phosphatidylcholine (POPC), 2 g of distearoylphosphatidylethanolamine-polyethylene glycol 2000 (DSPE-PEG2000), 2.5 g of cholesterol and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a 0.45 $\mu$m filter membrane to remove large particles, then sterilized by filtration through a 0.22 $\mu$m microporous filter membrane, so as to obtain the liposome, and encapsulation efficiency, particle size, PDI, and Zeta potential were measured. Results are shown in Table 1.

**Example 7**

A deoxycholic acid liposome was prepared (the first phospholipid: DMPC), with a mass ratio of deoxycholic acid, the first phospholipid, and the second phospholipid of 1:5:0.4.

5 g of deoxycholic acid, 25 g of dimyristoylphosphatidylcholine (DMPC), 2 g of distearoylphosphatidylethanolamine-polyethylene glycol 2000 (DSPE-PEG2000), 2.5 g of cholesterol and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a 0.45 $\mu$m filter membrane to remove large particles, then sterilized by filtration through a 0.22 $\mu$m microporous filter membrane, so as to obtain the liposome, and encapsulation efficiency, particle size, PDI, and Zeta potential were measured. Results are shown in Table 1.

**Example 8**

A deoxycholic acid liposome was prepared (the first phospholipid: DPPC), with a mass ratio of deoxycholic acid, the first phospholipid, and the second phospholipid of 1:5:0.4.

5 g of deoxycholic acid, 25 g of dipalmitoylphosphatidylcholine (DPPC), 2 g of distearoylphosphatidylethanolamine-polyethylene glycol 2000 (DSPE-PEG2000), 2.5 g of cholesterol and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a 0.45 μm filter membrane to remove large particles, then sterilized by filtration through a 0.22 μm microporous filter membrane, so as to obtain the liposome, and encapsulation efficiency, particle size, PDI, and Zeta potential were measured. Results are shown in Table 1.

## Example 9

A deoxycholic acid liposome was prepared (the first phospholipid: DOPC), with a mass ratio of deoxycholic acid, the first phospholipid, and the second phospholipid of 1:5:0.4.

5 g of deoxycholic acid, 25 g of dioleoylphosphatidylcholine (DOPC), 2 g of distearoylphosphatidylethanolamine-polyethylene glycol 2000 (DSPE-PEG2000), 2.5 g of cholesterol and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a 0.45 μm filter membrane to remove large particles, then sterilized by filtration through a 0.22 μm microporous filter membrane, so as to obtain the liposome, and encapsulation efficiency, particle size, PDI, and Zeta potential were measured. Results are shown in Table 1.

## Example 10

Preparation of deoxycholic acid liposome lyophilized product

Sucrose was added in a sample obtained after the homogenization step in Example 9 and completely dissolved, the volume was made up to 500 mL with a 0.025 M disodium succinate buffer solution containing 0.5% NaCl at pH 6-6.5, causing the content of sucrose in the solution to be 6%(w/w), the sample was first filtered through a 0.45 μm filter membrane to remove large particles, and then sterilized by filtration through a 0.22 μm microporous filter membrane, so as to obtain the liposome; and the liposome was filled in 6 mL vials, with a filling capacity of 2 mL/vial, and freeze drying was performed to obtain a deoxycholic acid liposome lyophilized product. The lyophilized product was redissolved with 2 mL of water for injection, and encapsulation efficiency, particle size, PDI, and Zeta potential were measured. Results are shown in Table 1.

## Example 11

Preparation of blank liposome (the first phospholipid: a blank liposome of DOPC)

25 g of dioleoylphosphatidylcholine (DOPC), 2 g of distearoylphosphatidylethanolamine-polyethylene glycol 2000 (DSPE-PEG2000), 2.5 g of cholesterol, and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a 0.45 μm filter membrane to remove large particles, then sterilized by filtration through a 0.22 μm microporous filter membrane, so as to obtain the blank liposome, and particle size, PDI, and Zeta potential were measured. Results are shown in Table 1.

**Example 12**

A deoxycholic acid liposome was prepared, with a mass ratio of deoxycholic acid, first phospholipid, and the second phospholipid of 1:3:0.4.

5 g of deoxycholic acid, 15 g of dioleoylphosphatidylcholine (DOPC), 2 g of distearoylphosphatidylethanolamine-polyethylene glycol 2000 (DSPE-PEG2000), 2.5 g of cholesterol, and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a 0.45 μm filter membrane to remove large particles, then sterilized by filtration through a 0.22 μm microporous filter membrane, so as to obtain the liposome, physical properties were observed, and encapsulation efficiency, particle size, PDI, and Zeta potential were measured. Results are shown in Table 2.

**Example 13**

A deoxycholic acid liposome was prepared, with a mass ratio of deoxycholic acid, first phospholipid, and the second phospholipid of 1:4:0.4.

5 g of deoxycholic acid, 20 g of dioleoylphosphatidylcholine (DOPC), 2 g of distearoylphosphatidylethanolamine-polyethylene glycol 2000 (DSPE-PEG2000), 2.5 g of cholesterol, and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a 0.45 μm filter membrane to remove large particles, then sterilized by filtration through a 0.22 μm microporous filter membrane, so as to obtain the liposome, physical properties were observed, and encapsulation efficiency, particle size, PDI, and Zeta potential were measured. Results are shown in Table 2.

**Example 14**

A deoxycholic acid liposome was prepared, with a mass ratio of deoxycholic acid, first phospholipid, and the second phospholipid of 1:6:0.4.

5 g of deoxycholic acid, 30 g of dioleoylphosphatidylcholine (DOPC), 2 g of distearoylphosphatidylethanolamine-polyethylene glycol 2000 (DSPE-PEG2000), 2.5 g of cholesterol, and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a 0.45 μm filter membrane to remove large particles, then sterilized by filtration through a 0.22 μm microporous filter membrane, so as to obtain the liposome, physical properties were observed, and encapsulation efficiency, particle size, PDI, and Zeta potential were measured. Results are shown in Table 2.

**Example 15**

A deoxycholic acid liposome was prepared (excluding DSPE-PEG2000), with a mass ratio of deoxycholic acid, first phospholipid, and the second phospholipid of 1:5:0.

5 g of deoxycholic acid, dioleoylphosphatidylcholine (DOPC), 2.5 g of cholesterol, and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a 0.45 μm filter membrane to remove large particles, then sterilized by filtration through a 0.22 μm microporous filter membrane, so as to obtain the liposome, physical properties were observed, and encapsulation efficiency, particle size, PDI, and Zeta potential were measured. Results are shown in Table 2.

**Example 16**

A deoxycholic acid liposome was prepared, with a mass ratio of deoxycholic acid, first phospholipid, and the second phospholipid of 1:5:0.4.

5 g of deoxycholic acid, 25 g of dioleoylphosphatidylcholine (DOPC), 2 g of distearoylphosphatidylethanolamine-polyethylene glycol 2000 (DSPE-PEG2000), 2.5 g of cholesterol and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M histidine solution was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M histidine solution, the obtained sample was first filtered through a 0.45 μm filter membrane to remove large particles, then sterilized by filtration through a 0.22 μm microporous filter membrane, so as to obtain the liposome, physical properties were observed, and encapsulation efficiency, particle size, PDI, and Zeta potential were measured. Results are shown in Table 2.

**Example 17**

A deoxycholic acid liposome was prepared, with a mass ratio of deoxycholic acid, first phospholipid, and the second phospholipid of 1:5:0.4.

5 g of deoxycholic acid, 25 g of dioleoylphosphatidylcholine (DOPC), 2 g of distearoylphosphatidylethanolamine-polyethylene glycol 2000 (DSPE-PEG2000), 2.5 g of cholesterol and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.05M disodium succinate buffer solution was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.05M disodium succinate buffer solution, the obtained sample was first filtered through a 0.45 μm filter membrane to remove large particles, then sterilized by filtration through a 0.22 μm microporous filter membrane, so as to obtain the liposome, physical properties were observed, and encapsulation efficiency, particle size, PDI, and Zeta potential were measured. Results are shown in Table 2.

**Example 18** Preparation of reference formulation (Kybella) (100 mL)

900 mg of benzyl alcohol, 1000 mg of deoxycholic acid, 142 mg of sodium dihydrogen phosphate, 143 mg of sodium hydroxide, and 438 mg of sodium chloride were weighed and fully dissolved in 80 mL of water for injection, then pH was adjusted to 8.3 with 1M sodium hydroxide solution, and the solution was finally made up to 100 mL with water for injection, and the reference formulation was obtained after sterilization by filtration through a 0.2 μm filter. The active ingredient concentration of the obtained deoxycholic acid solution was 10 mg/mL.

**Example 19**

A deoxycholic acid liposome was prepared, with a mass ratio of deoxycholic acid, first phospholipid, and the second phospholipid of 1:30:0.4.

1 g of deoxycholic acid, 30 g of dioleoylphosphatidylcholine (DOPC), 0.4 g of distearoylphosphatidylethanolamine-polyethylene glycol 2000 (DSPE-PEG2000), 0.5 g of cholesterol and 0.125 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, 100 mL was made up with the 0.025 M disodium succinate buffer solution containing 0.5% NaCl at pH 6-6.5, the obtained sample was first filtered through a (1.0+0.65) $\mu$m double-layer filter membrane to remove large particles, then filtration sterilization was performed through a 0.22 $\mu$m microporous filter membrane, so as to obtain the liposome, physical properties were observed, and encapsulation efficiency, particle size, PDI, and Zeta potential were measured. Results are shown in Table 2.

**Example 20**

A deoxycholic acid liposome was prepared (the second phospholipid: DSPG), with a mass ratio of deoxycholic acid, first phospholipid, and the second phospholipid of 1:5:0.4.

5 g of deoxycholic acid, 25 g of dioleoylphosphatidylcholine (DOPC), 2 g of 1,2-distearoyl-sn-glycero-3-phosphocholine (DSPG), 2.5 g of cholesterol and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a (1.0+0.65) $\mu$m double-layer filter membrane to remove large particles, then filtration sterilization was performed through a 0.22 $\mu$m microporous filter membrane, so as to obtain the liposome, physical properties were observed, and encapsulation efficiency, particle size, PDI, and Zeta potential were measured. Results are shown in Table 2.

**Example 21**

A deoxycholic acid liposome was prepared (the second phospholipid: DSPE-PEG2000), with a mass ratio of deoxycholic acid, first phospholipid, and the second phospholipid of 1:5:0.5.

5 g of deoxycholic acid, 25 g of dioleoylphosphatidylcholine (DOPC), 2.5 g of distearoylphosphatidylethanolamine-polyethylene glycol 2000 (DSPE-PEG2000), 2.5 g of cholesterol and 0.625 g of vitamin E acetate were weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a (1.0+0.65) $\mu$m double-layer filter membrane to remove large particles, then filtration sterilization was performed through a 0.22 $\mu$m microporous filter membrane, so as to obtain the liposome, physical properties were observed, and encapsulation efficiency, particle size, PDI, and Zeta potential were measured. Results are shown in Table 2.

**Example 22**

A deoxycholic acid liposome was prepared (excluding cholesterol), with a mass ratio of deoxycholic acid, first phospholipid, and the second phospholipid of 1:5:0.4.

5 g of deoxycholic acid, 25 g of dioleoylphosphatidylcholine (DOPC), 2 g of distearoylphosphatidylethanolamine-polyethylene glycol 2000 (DSPE-PEG2000), 0.625 g of vitamin E acetate was weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a (1.0+0.65) $\mu$m double-layer filter membrane to remove large particles, then sterilized by filtration through a 0.22 $\mu$m microporous filter membrane, so as to obtain the liposome, physical properties were observed, and encapsulation efficiency, particle size, PDI, and Zeta potential were measured. Results are shown in Table 2.

**Example 23**

A deoxycholic acid liposome was prepared (excluding vitamin E acetate), with a mass ratio of deoxycholic acid, first phospholipid, and the second phospholipid of 1:5:0.4.

5 g of deoxycholic acid, 25 g of dioleoylphosphatidylcholine (DOPC), 2 g of distearoylphosphatidylethanolamine-polyethylene glycol 2000 (DSPE-PEG2000), 2.5 g of cholesterol was weighed and mixed, the mixture was dissolved in a mixed organic solvent of trichloromethane and anhydrous methanol (v/v=10:1) to prepare an organic phase with a total solid content of 20%(w/v), and an organic solvent was removed by vacuum evaporation at 45-60 °C, so as to obtain a lipid film. Then, 450 mL of a 0.025 M disodium succinate buffer solution containing 0.5% NaCl at pH 6-6.5 was added to the above prepared film for hydration with a hydration temperature of 20-25 °C and a hydration time of 60-120 min, homogenization was performed three times under a homogenization condition of 8000-12000 psi after complete hydration, the volume was made up to 500 mL with the 0.025 M disodium succinate buffer solution containing 0.5% NaCl at pH 6-6.5, the obtained sample was first filtered through a (1.0+0.65) $\mu$m double-layer filter membrane to remove large particles, then sterilized by filtration through a 0.22 $\mu$m microporous filter membrane, so as to obtain the liposome, physical properties were observed, and encapsulation efficiency, particle size, PDI, and Zeta potential were measured. Results are shown in Table 2.

Although the above describes specific implementations of the present invention, those skilled in the art should understand that these are merely illustrative examples. Various changes or modifications may be made to these implementations without departing from the principles and spirit of the present invention. Therefore, the scope of protection of the present invention is defined by the appended claims.

**Test example 1 Example sample quality detection**

Physical property detection: the sample prepared in the above examples was taken, physical properties were observed, and test results are shown in Table 1 and Table 2.

Detection of particle size and PDI: 25 $\mu$L of the liposome sample prepared in the examples was taken and diluted to 5 mL with 0.9% NaCl, the particle size and PDI of the taken sample were measured, and test results are shown in Table 1 and Table 2.

Zeta potential detection: 25 $\mu$L of the liposome sample prepared in the examples was taken and diluted to 5 mL with water for injection, Zeta potential of the taken sample was measured, and test results are shown in Table 1 and Table 2.

Table 1 Quality detection results of liposome

| Example | encapsulation efficiency (%) | Particle size (nm) | PDI | Zeta potential (mV) |
|---|---|---|---|---|
| Example 1 (Comparative example) | 59.62 | 96.05 | 0.225 | -25.6 |
| Example 2 (first phospholipid SPC) | 78.96 | 91.30 | 0.198 | -28.4 |
| Example 3 (first phospholipid HSPC) | 75.62 | 294.33 | 0.616 | -29.5 |
| Example 4 (first phospholipid DSPC) | 72.60 | 132.12 | 0.205 | -28.2 |
| Example 5 (first phospholipid DEPC) | 94.23 | 90.71 | 0.173 | -39.8 |
| Example 6 (first phospholipid POPC) | 93.57 | 94.42 | 0.184 | -39.2 |
| Example 7 (first phospholipid DMPC) | 72.65 | 134.6 | 0.321 | -32.6 |
| Example 8 (first phospholipid DPPC) | 79.62 | 141.2 | 0.264 | -31.2 |
| Example 9 (first phospholipid DOPC) | 98.99 | 62.45 | 0.189 | -40.3 |
| Example 10* | 98.86 | 68.46 | 0.185 | -40.4 |
| Example 11* | N/A | 90.23 | 0.182 | -30.6 |

*Note: Example 10 used a lyophilized preparation of a deoxycholic acid liposome, the above quality investigation was performed after redissolving it in 2 mL of water for injection; and Example 11 used a blank liposome with the first phospholipid DOPC. N/A indicates not applicable.

Investigation results of Table 1 above showed that, the encapsulation efficiency of the liposome prepared in Example 1 (Comparative example) was very low; and the encapsulation efficiency of the liposomes prepared in Example 2, Example 3, Example 4, Example 7, and Example 8 was lower than 80%. Furthermore, the particle sizes of the liposomes prepared in Examples 3-4 and 7-8 were all greater than 100 nm. The liposomes prepared according to the four formulations in Example 5, Example 6, Example 9, and Example 10 all had the encapsulation efficiency greater than 90% and the particle sizes less than 100 nm, and thus had good dispersion characteristics, the potential for uniform lipolysis, higher Zeta potentials, and good stability.

Table 2 Quality detection results of liposome

| Example | Physical property | encapsulation efficiency (%) | Particle size (nm) | PDI | Zeta potential (mV) |
|---|---|---|---|---|---|
| Example 12 | White suspension | 75.18 | 142.78 | 0.326 | -39.5 |
| Example 13 | Translucent white suspension | 88.32 | 70.76 | 0.199 | -39.7 |
| Example 9 | Translucent white suspension | 98.99 | 62.45 | 0.189 | -40.3 |
| Example 14 | Translucent white suspension | 98.89 | 62.35 | 0.191 | -40.1 |
| Example 15 (without DSPE-PEG2000) | Translucent white suspension | 90.68 | 65.35 | 0.189 | -8.6 |
| Example 16 (an aqueous phase being a histidine solution) | Translucent white suspension | 85.62 | 89.56 | 0.215 | -38.7 |
| Example 17 (an aqueous phase being a disodium succinate buffer solution) | Translucent white suspension | 87.35 | 86.27 | 0.226 | -39.2 |
| Example 18 (Reference formulation) | Uniform, transparent liquid | N/A | N/A | N/A | N/A |
| Example 19 | Translucent white suspension | 92.10 | 67.50 | 0.196 | -39.5 |
| Example 20 (the second phospholipid DSPG) | Translucent white suspension | 94.30 | 86.89 | 0.187 | -38.6 |
| Example 21 | Translucent white suspension | 98.0 | 90.94 | 0.281 | -45.3 |
| Example 22 (excluding cholesterol) | Translucent white suspension | 95.5 | 95.54 | 0.330 | -37.8 |
| Example 23 (excluding vitamin E acetate) | Translucent white suspension | 90.3 | 93.25 | 0.310 | -38.8 |

Investigation results of Table 2 showed that, in Example 12 (a mass ratio of the deoxycholic acid to the first phospholipid being 1:3), Example 9 (1:5), Example 13 (1:4), and Example 14 (1:6), when the mass ratio of the deoxycholic acid to the dioleoylphosphatidylcholine (DOPC) was 1:3 (Example 12), the suspension had poor physical properties and was not opalescent, the encapsulation efficiency was lower than 80%, the liposome had poor quality, the particle size and the PDI were relatively large, and product dispersion characteristics were also poor. The liposome samples in Example 9, Example 13, and Example 14 had a small quality difference, that is, when the mass ratio of the deoxycholic acid to the dioleoylphosphatidylcholine (DOPC) was 1:4, 1:5, or 1:6, the liposome had high encapsulation efficiency and small particle size and PDI, more facilitating releasing of the product in the body. When the mass ratio of the deoxycholic acid to the dioleoylphosphatidylcholine (DOPC) was greater than 1:6, that is, the liposome prepared in Example 19 (1:30) had high encapsulation efficiency and small particle size and PDI, and thus the liposome had excellent quality. When the amount of the first phospholipid dioleoylphosphatidylcholine (DOPC) was further increased, the encapsulation efficiency, particle size, and PDI of the liposome did not undergo significant changes. Considering the economic costs and safety risks posed by excipients to patients, it was not recommended to further increase the dosage ratio of the phospholipid.

From the investigation results in Table 2, it was further observed that when the formula did not contain the second phospholipid distearoylphosphatidylethanolamine-polyethylene glycol 2000 (DSPE-PEG2000) (Example 15), although the physical properties, encapsulation efficiency, particle size, and PDI of the liposome suspension showed no significant difference compared to that containing the second phospholipid, the Zeta potential was lower than -10 mV, indicating that the stability of the product was poor. For the liposomes prepared in Example 9, Example 13, and Example 14, the Zeta potential was maintained at about -40 mV, indicating that the stability of the product was good. Compared with Example 9, when the amount of the second phospholipid distearoylphosphatidylethanolamine-polyethylene glycol 2000 (DSPE-PEG2000) (Example 21) increased, an absolute value of the Zeta potential increased, facilitating liposome stability.

From the investigation results in Table 2, it was further observed that when the formula contained the second phospholipid 1,2-distearoyl-sn-glycero-3-phosphocholine (DSPG) (Example 20), the encapsulation efficiency was high, and the particle size and the PDI were small, facilitating the dispersion stability of the product.

From the investigation results in Table 2, it was further observed that, when the 0.025M histidine solution (Example 16), the 0.05M disodium succinate buffer solution (Example 17), and the 0.025 M disodium succinate buffer solution containing 0.5% NaCl at pH 6-6.5 were used as the aqueous phases, the prepared liposomes all had high encapsulation efficiency, especially the liposome prepared in Example 9, which has small particle size and PDI, facilitating good dispersibility.

From the investigation of Table 2, it was further observed that, when the formula did not contain cholesterol (Example 22) or vitamin E acetate (Example 23), the quality attributes of the prepared liposomes, such as encapsulation efficiency, particle size, and PDI, showed no significant difference.

**Test example 2 Investigation of killing capability Against adipocytes**

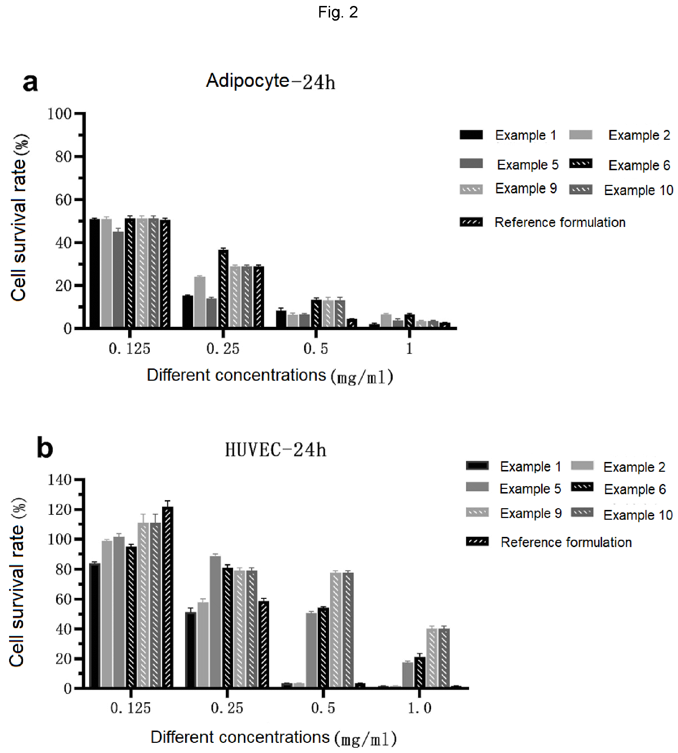

Experimental Protocol: 3T3-L1 (mouse embryonic fibroblasts) was inoculated into a 96-well plate in advance and used for subsequent cell evaluation after being subjected to induced differentiation to form mature adipocytes, the mature adipocytes and Human Umbilical Vein Endothelial Cells (HUVECs) were added to the 96-well plate at a count of 10,000 cells per well, with a volume of 100 $\mu$L per well, and the 96-well plate was put in a carbon dioxide incubator for incubation and used for cell evaluation after 24 h of cell culture. A complete medium was used to respectively dilute the samples of Example 18 and Examples 1-10 to concentrations of the deoxycholic acid of 1 mg/mL, 0.5 mg/mL, 0.25 mg/mL, and 0.125 mg/mL with water for injection. The lyophilized sample of Example 10 was redissolved with water for injection, and then diluted to the above concentrations with water for injection. The sample of Example 11 (blank liposome) was diluted at the same ratio by referring to the corresponding drug-loaded liposomes, so as to obtain the blank liposome solution. The above drug-loaded liposomes with different deoxycholic acid concentrations, the reference formulation, and the blank liposome solution were respectively added to the cell plate of the mature adipocytes and the HUVEC (n=4), with 100 $\mu$L per cell. After administration, the plate was continuously cultured for 24 h in the carbon dioxide incubator, 100 $\mu$L of supernatant in each well was discarded, 50 $\mu$L of a cell viability detection reagent (Cell Counting-Lite 3D Luminescent Cell Viability Assay) was added, oscillation and well mixing were performed at room temperature for 5 min until cell masses were completely lysed. After incubation for 30 min, 50 $\mu$L of the mixture was transferred to an opaque 96-well plate (50 $\mu$L per well). Fluorescence Intensity (FI) was measured by a fluorescent microplate reader, and the cell survival rate was calculated according to the following formula. Cell killing results were fitted and counted by Graphpad software according to the cell survival rate, and IC50 was calculated. Cell survival rate experiment results are shown in Fig. 2, and cell killing capability experiment results are shown in Table 3.

$$\text{Cell survival rate (\%)} = (\text{FI sample} - \text{FI blank})/(\text{FI control} - \text{FI blank}) \times 100\%$$

FI blank: the mean fluorescence intensity measured in the absence of cells and without any sample treatment.

FI control: the mean fluorescence intensity measured in the presence of cells but without any sample treatment.

Table 3 Killing capability to different cells (IC50) (Unit: mg/mL)

| Sample group | Adipocyte | HUVEC |
|---|---|---|
| Example 18 (Reference formulation) | 0.13 | 0.28 |
| Example 1 (Comparative example) sample | 0.13 | 0.27 |
| Example 2 sample | 0.13 | 0.30 |
| Example 3 sample | 0.14 | 0.43 |
| Example 4 sample | 0.14 | 0.41 |
| Example 5 sample | 0.12 | 0.56 |
| Example 6 sample | 0.13 | 0.58 |
| Example 7 sample | 0.12 | 0.49 |
| Example 8 sample | 0.13 | 0.52 |
| Example 9 sample | 0.13 | 0.86 |
| Example 10 sample | 0.13 | 0.84 |

From Table 3, it was observed that after 24 h of administration, Example 11 (blank liposome) had no killing capability against rat adipocytes and non-adipocyte HUVEC (Human Umbilical Vein Endothelial Cells). The killing capabilities (IC50) of samples to be tested in other groups againstrat adipocytes were essentially comparable. However, the killing capabilities (IC50) of the liposomes in Example 9 and Example 10 against non-adipocyte HUVEC (Human Umbilical Vein Endothelial Cells) were significantly weaker than those of the reference formulation (Example 18) and Example 1 (Comparative example), and also weaker than those of Examples 2-8. Therefore, the liposomes in Example 9 and Example 10 had excellent adipocyte selectivity.

From the above table, it was also be observed that the liposome prepared in Example 9 and the liposome lyophilized sample prepared in Example 10 had nearly identical killing capabilities against the rat adipocytes and the HUVEC cells. Therefore, the deoxycholic acid liposomes did not bring adverse effects on product quality or therapeutic efficacy after being prepared into the lyophilized product, and they could be further prepared into the lyophilized preparations.

From Fig. 2, it was observed that as the administration concentration of the deoxycholic acid increased, the cell survival rates of the adipocytes and non-adipocyte HUVEC cells in each test group further reduced. When the administration concentrations of the deoxycholic acid were 0.125 mg/mL and 0.25 mg/mL, the survival rate of the non-adipocyte HUVEC cells in each test group was higher than the survival rate of the adipocytes at the same administration concentration. When the administration concentration of the deoxycholic acid was 0.5 mg/mL, the survival rates of the adipocytes and non-adipocyte HUVEC cells of Example 1 (Comparative example), Example 2, and a reference formulation (Example 18) treatment group all reduced below 10%, and the non-adipocyte survival rates in groups of Example 5, Example 6, Example 9, and Example 10 could maintain above 50%, in particular, the non-adipocyte survival rates in the groups of Example 9 and Example 10 nearly reached 80%. When the administration concentration of the deoxycholic acid was 1.0 mg/mL, Example 5, Example 6, Example 9, and Example 10 also showed weaker non-adipocyte killing capability compared to Example 1 (Comparative example), Example 2, and the reference formulation (Example 18). Therefore, Example 5, Example 6, Example 9, and Example 10 had excellent adipocyte selectivity.

**Test example 3 Liposome shape testing**

The liposome lyophilized sample prepared in Example 10 was taken, 2 mL of water for injection was added for redissolution, the shape of the liposome was observed by a cryo-transmission electron microscopy, 5 $\mu$l of the liposome was accurately weighed and placed on a R1.2/1.3 100-well carbon film grid (Cu 200 mesh), excess liposome suspension was rapidly blotted, and the carbon film grid was frozen in a mixture of liquid ethane and methane. An FEI Talos F200C electron microscope was used to obtain data at an operating voltage of 200 kV, and the collected data was processed by using SerialEM software.

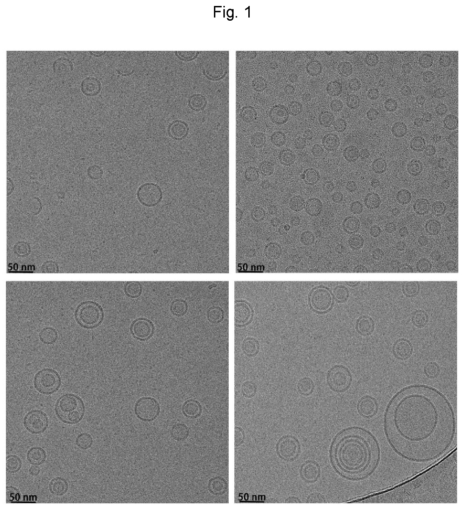

Through cryo-transmission electron microscopy, the liposome lyophilized sample was observed to be a liposome having a complete vesicular structure, instead of a lipid complex. Furthermore, under the electron microscopy, most of these liposomes were observed to adopt a double-chambered conformation, while a minority showed a multi-chambered conformation, as shown in Fig. 1.

**Test example 4 Deoxycholic acid liposome stability investigation**

The liposome lyophilized sample prepared in Example 10 was taken and placed in a 2-8 °C stability chamber; the encapsulation efficiency, particle size, and PDI were measured by taking samples in Month 0, Month 1, Month 2, Month 3, and Month 6; and the detection methods were the same as Test example 1. Results are shown in Table 4.

Table 4 Liposome stability investigation data

| Storage time | encapsulation efficiency (%) | Particle size (nm) | PDI |
|---|---|---|---|
| Month 0 | 98.86 | 68.46 | 0.185 |
| Month 1 | 98.92 | 70.72 | 0.186 |
| Month 3 | 98.93 | 72.26 | 0.176 |
| Month 6 | 99.32 | 73.86 | 0.182 |

The above investigation results showed that the liposome lyophilized preparation prepared in Example 10 maintained an essentially unchanged encapsulation efficiency after 6 months of storage (data variation within the 2% detection error range) and showed no significant change in particle size, indicating excellent stability and quality.

**Test example 5 Investigation of lipolysis effect of liposomes on tail adipose of ZDF rats**

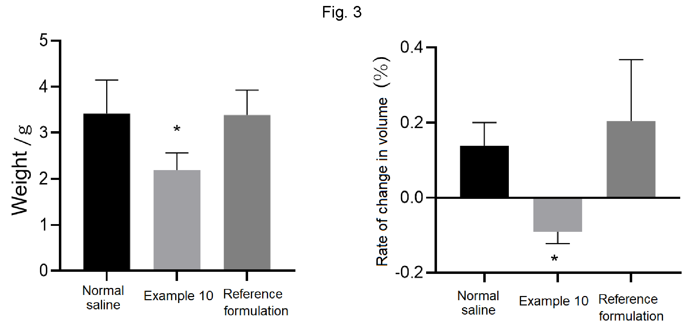

Twelve male ZDF rats weighting 330-350 g were selected and divided into 3 groups randomly: a normal saline group, DC-LIP (sample prepared in Example 10), and the reference formulation (Example 18), with 4 rats per group. The rats were anesthetized with isoflurane, tail adipose volumes were measured by using Micro-CT before administration, and reconstruction was performed to calculate an initial tail adipose volume. Then, 2.5 mL of the sample to be tested was injected in tail adipose tissue on both sides of the ZDF rat, respectively. The sample of Example 10 was the lyophilized preparation, which was redissolved with 2 mL of water for injection, where the concentration of the deoxycholic acid was 10 mg/mL, and the drug concentration of the reference formulation was 10 mg/mL. The tail adipose tissue was detected again by Micro-CT 2 weeks after administration, so as to calculate the post-administration volume. After the last CT detection, tail adipose was weighed, and the weights of the groups were compared.
The lipolysis efficacy of the sample to be tested was demonstrated by the rate of change in adipose volume and the reduction in adipose weight before and after administration. Rate of change in adipose volume (%) = [(adipose volume after administration-adipose volume before administration)/adipose volume before administration]*100%. Experiment results are shown in Fig. 3.
From Fig. 3, it was observed that, 2 weeks after administration, the tail adipose volume in the normal saline group increased by 13.8%, the tail adipose volume in the DC-LIP group (Example 10) was reduced by 9%, and the tail adipose volume of the reference formulation group increased by 20.5%. For the weight of the tail adipose, compared to the normal saline group and the reference formulation group, the weight of the tail adipose in the ZDF rat in the DC-LIP group (Example 10) was significantly reduced. Compared to the normal saline group, the adipose volume and weight of the reference formulation group were increased, and during autopsy, the injected adipose tissue showed significant signs of necrosis, such as a grayish-brown discoloration of the adipose tissue, indicating that the reference formulation had exerted its pharmacological effect of lysing adipose tissue at the injection site. However, due to the indiscriminate lysis of cell membranes by the reference formulation, it caused a relatively severe inflammatory reaction at the injection site, resulting in an increase in adipose volume and weight.
While evaluating the in vivo lipolysis effect, adverse reactions at the injection sites were observed, as shown in Table 5. During adverse reaction monitoring, no swelling, induration, or ulceration was observed in any group on Day 1 after administration. On Day 7 after administration, the DC-LIP group (Example 10) showed only very minimal swelling (+), with no induration or skin ulceration observed, and swelling, induration, and skin ulceration were present in all the reference formulation groups. On Day 14 after administration, swelling disappeared in the DC-LIP group (Example 10), while swelling, induration, and ulceration were still present in the reference formulation group. No swelling, induration, or skin ulceration was observed in the normal saline control group. The adverse reactions in the DC-LIP group were milder.

Table 5 Incidence of injection site adverse reactions

| Administration time | Swelling | | Induration | | Ulceration | |
|---|---|---|---|---|---|---|
| | Example 9 | Reference formulation | Example 9 | Reference formulation | Example 9 | Reference formulation |
| Day 1 | 0% (0/8) | 100% (8/8) ++ | 0% (0/8) | 0% (0/8) | 0% (0/8) | 0% (0/8) |
| Day 7 | 100% (8/8) + | 100% (8/8) +++ | 0% (0/8) | 100% (8/8) +++ | 0% (0/8) | 100% (8/8) |
| Day 14 | 0% (0/8) | 100% (6/8) +++ | 0% (0/8) | 100% (8/8) +++ | 0% (0/8) | 100% (8/8) |

Note: ① Percentage incidence (%), with data in parentheses indicating the number of adverse reaction cases and total cases; ② swelling severity was semi-quantitatively described using a five point method, such as Grade 0, Grade 1 (+), Grade 2 (++), Grade 3 (+++), and Grade 4 (++++), with higher grades indicating more severe adverse reactions; ③ 0/8 indicated the number among 8 tail adipose pads showing swelling, induration, or ulceration in 4 animals of each group (each animal had 2 tail adipose pads).

The above tail lipolysis and local adverse reaction results showed that the drug-loaded liposomes in Example 10 could not only show the faster lipolysis effect but also mitigate the local adverse reactions of the reference formulation.

**Claims**

1. A liposome drug delivery system, wherein the liposome drug delivery system comprises deoxycholic acid and phospholipid, the phospholipid comprises a first phospholipid and optionally comprises a second phospholipid; wherein the mass ratio of the deoxycholic acid to the phospholipid is 1:2.5-1:35.

2. The liposome drug delivery system according to claim 1, wherein, the mass ratio of the deoxycholic acid to the phospholipid is 1:3.4-1:30.4, such as 1:3.4, 1:3.6, 1:4.4, 1:5, 1:5.4, 1:5.5, 1:6, 1:6.4, or 1:30.4, preferably 1:4.4-1:30.4.

3. The liposome drug delivery system according to at least one of claims 1 to 2, wherein, the first phospholipid is selected from one or more of egg yolk lecithin (PC), soy lecithin (SPC), hydrogenated soy lecithin (HSPC), dioleoylphosphatidylcholine (DOPC), dierucoylphosphatidylcholine (DEPC), distearylphosphatidylcholine (DSPC), 1-palmitoyl-2-oleoyl-phosphatidylcholine (POPC), dimyristoylphosphatidylcholine (DMPC), dipalmitoylphosphatidylcholine (DPPC), distearoylphosphatidylethanolamine (DSPE), and dioleylphosphatidylethanolamine (DOPE), preferably soy lecithin (SPC), hydrogenated soy lecithin (HSPC), distearylphosphatidylcholine (DSPC), dimyristoylphosphatidylcholine (DMPC), dipalmitoylphosphatidylcholine (DPPC), dioleoylphosphatidylcholine (DOPC), dierucoylphosphatidylcholine (DEPC), and 1-palmitoyl-2-oleoyl-phosphatidylcholine (POPC), more preferably soy lecithin (SPC), dipalmitoylphosphatidylcholine (DPPC), dioleoylphosphatidylcholine (DOPC), dierucoylphosphatidylcholine (DEPC), or 1-palmitoyl-2-oleoyl-phosphatidylcholine (POPC), more preferably dioleoylphosphatidylcholine (DOPC), dierucoylphosphatidylcholine (DEPC), or 1-palmitoyl-2-oleoyl-phosphatidylcholine (POPC), such as dioleoylphosphatidylcholine (DOPC); and/or
   the second phospholipid is selected from one or more of 1,2-distearoyl-sn-glycero-3-phosphocholine (DSPG), dioleoylphosphatidylserine (DOPS), distearoylphosphatidylserine (DSPS), (2-dioleoyl hydroxypropyl)trimethylammonium chloride (DOTAP), 1,2-dioleyloxy-3-dimethylamino-propane (DODMA), distearoylphosphatidylethanolamine-polyethylene glycol 2000 (DSPE-PEG2000), dioleoylphosphatidylglycerol (DOPG), and egg yolk phosphatidylglycerol (EPG), preferably distearoylphosphatidylethanolamine-polyethylene glycol 2000 (DSPE-PEG2000), 1,2-distearoyl-sn-glycero-3-phosphocholine (DSPG), dioleoylphosphatidylglycerol (DOPG), or 1,2-dioleyloxy-3-dimethylamino-propane (DODMA), preferably distearoylphosphatidylethanolamine-polyethylene glycol 2000 (DSPE-PEG2000) or 1,2-distearoyl-sn-glycero-3-phosphocholine (DSPG), more preferably distearoylphosphatidylethanolamine-polyethylene glycol 2000 (DSPE-PEG2000).

4. The liposome drug delivery system according to at least one of claims 1 to 3, wherein, the mass ratio of the deoxycholic acid to the first phospholipid is 1:3-1:32, preferably 1:4-1:30, more preferably 1:4-1:6, such as 1:4, 1:5, or 1:6; and/or

   the mass ratio of the deoxycholic acid, the first phospholipid, and the second phospholipid is 1:(3-32):(0-3), preferably, the mass ratio of the deoxycholic acid, the first phospholipid, and the second phospholipid is 1:(4-30):(0.2-0.6), more preferably 1:3:0.4, 1:4:0.4, 1:5:0.4, 1:5:0.5, 1:6:0.4, or 1:30:0.4; and/or
   the mass ratio of the first phospholipid to the second phospholipid is (3-32):(0-3), preferably, the mass ratio of the first phospholipid to the second phospholipid is (4-30):(0.2-0.6), more preferably 3:0.4, 4:0.4, 5:0.4, 5:0.5, 6:0.4, or 30:0.4; and/or
the ratio of drug to lipid is 1:(3-32), preferably 1:(4-30.9), such as 1:5.9, 1:3.9, 1:4.0, 1:4.9, 1:6.9, 1:5.5, 1:6, or 1:6.1.

5. The liposome drug delivery system according to at least one of claims 1 to 4, wherein, the mass ratio of the deoxycholic acid to the phospholipid is 1:4-1:7, preferably 1:4.3-1:6.5, more preferably 1:(5-6); and/or
the mass ratio of the deoxycholic acid, the first phospholipid, and the second phospholipid is 1:(4-10):(0-0.6), preferably 1:(4-6):(0.2-0.6), more preferably 1:(4-6):(0.4-0.5).

6. The liposome drug delivery system according to at least one of claims 1 to 5, wherein, the liposome drug delivery system optionally comprises cholesterol and/or an antioxidant, preferably, the antioxidant is selected from vitamin E and/or vitamin E acetate.

7. The liposome drug delivery system according to at least one of claims 1 to 6, wherein, the liposome drug delivery system further comprises an aqueous phase, wherein the aqueous phase is selected from one or more of a disodium succinate buffer solution, a histidine solution, normal saline, a sucrose solution, and water for injection;

   preferably, the aqueous phase is selected from one or more of a 0.02-0.04 M disodium succinate buffer solution containing 0.5% NaCl, a 0.05 M disodium succinate buffer solution, a 0.025 M histidine solution, normal saline, and an 8% sucrose solution; more preferably, the aqueous phase is the 0.02-0.04 M disodium succinate buffer solution containing 0.5% NaCl; and
   more preferably, the pH value of the 0.02-0.04 M disodium succinate buffer solution containing 0.5% NaCl is 5.0-7.0; and preferably, the pH value is 6-6.5.

8. The liposome drug delivery system according to at least one of claims 1 to 7, wherein,

   each unit of the preparation is prepared from the following components: 1-5 parts of the deoxycholic acid, 15-30 parts of the first phospholipid, 0-2 parts of the second phospholipid, and 100-5000 parts of the aqueous phase, preferably 500-5000 parts of the aqueous phase; or each unit of the preparation is prepared from the following components: 1-5 parts of the deoxycholic acid, 15-30 parts of the first phospholipid, 0-2 parts of the second phospholipid, 500-5000 parts of the aqueous phase, 0-2.5 parts of cholesterol, and 0-0.625 parts of vitamin E acetate; and
   preferably, each unit of the preparation is prepared from the following components: 5 parts of the deoxycholic acid, 15-30 parts of the first phospholipid, 0-2 parts of the second phospholipid, 500 parts of the aqueous phase, 0-2.5 parts of cholesterol, and 0-0.625 parts of vitamin E acetate.

9. The liposome drug delivery system according to at least one of claims 1 to 8, wherein, the first phospholipid is selected from one or more of soy lecithin (SPC), dipalmitoylphosphatidylcholine (DPPC), dioleoylphosphatidylcholine (DOPC), dierucoylphosphatidylcholine (DEPC), and 1-palmitoyl-2-oleoyl-phosphatidylcholine (POPC); preferably, the first phospholipid is selected from one or more of dioleoylphosphatidylcholine (DOPC), dierucoylphosphatidylcholine (DEPC), and 1-palmitoyl-2-oleoyl-phosphatidylcholine (POPC), more preferably dioleoylphosphatidylcholine (DOPC);

   the second phospholipid is selected from one or more of 1,2-distearoyl-sn-glycero-3-phosphocholine (DSPG), dioleoylphosphatidylserine (DOPS), distearoylphosphatidylserine (DSPS), (2-dioleoyl hydroxypropyl)trimethylammonium chloride (DOTAP), 1,2-dioleyloxy-3-dimethylamino-propane (DODMA), distearoylphosphatidylethanolamine-polyethylene glycol 2000 (DSPE-PEG2000), dioleoylphosphatidylglycerol (DOPG), and egg yolk phosphatidylglycerol (EPG); preferably, the second phospholipid is selected from one or more of distearoylphosphatidylethanolamine-polyethylene glycol 2000 (DSPE-PEG2000) and 1,2-distearoyl-sn-glycero-3-phosphocholine (DSPG), more preferably distearoylphosphatidylethanolamine-polyethylene glycol 2000 (DSPE-PEG2000); and
   the aqueous phase is selected from one or more of the 0.02-0.04 M disodium succinate buffer solution containing 0.5% NaCl, the 0.05 M disodium succinate buffer solution, the 0.025 M histidine solution, the normal saline, and the 8% sucrose solution; preferably, the aqueous phase is one or more of the 0.02-0.04 M disodium succinate buffer solution containing 0.5% NaCl, the 0.025 M histidine solution, the normal saline, and the 8% sucrose solution; more preferably, the aqueous phase is the 0.02-0.04 M disodium succinate buffer solution containing 0.5% NaCl.

10. The liposome drug delivery system according to at least one of claims 1 to 9, wherein, the first phospholipid and the second phospholipid are selected from any one of the following combinations:

(1) soy lecithin (SPC) and distearoylphosphatidylethanolamine-polyethylene glycol 2000 (DSPE-PEG2000);
(2) hydrogenated soy lecithin (HSPC) and distearoylphosphatidylethanolamine-polyethylene glycol 2000 (DSPE-PEG2000);
(3) distearylphosphatidylcholine (DSPC), and distearoylphosphatidylethanolamine-polyethylene glycol 2000 (DSPE-PEG2000);
(4) dierucoylphosphatidylcholine (DEPC) and distearoylphosphatidylethanolamine-polyethylene glycol 2000 (DSPE-PEG2000);
(5) 1-palmitoyl-2-oleoyl-phosphatidylcholine (POPC) and distearoylphosphatidylethanolamine-polyethylene glycol 2000 (DSPE-PEG2000);
(6) dimyristoylphosphatidylcholine (DMPC) and distearoylphosphatidylethanolamine-polyethylene glycol 2000 (DSPE-PEG2000);
(7) dipalmitoylphosphatidylcholine (DPPC) and distearoylphosphatidylethanolamine-polyethylene glycol 2000 (DSPE-PEG2000);
(8) dioleoylphosphatidylcholine (DOPC) and distearoylphosphatidylethanolamine-polyethylene glycol 2000 (DSPE-PEG2000);
(9) dioleoylphosphatidylcholine (DOPC) and 1,2-distearoyl-sn-glycero-3-phosphocholine (DSPG);
(10) dioleoylphosphatidylcholine (DOPC) and dioleoylphosphatidylglycerol (DOPG); and
(11) dioleoylphosphatidylcholine (DOPC) and 1,2-dioleyloxy-3-dimethylamino-propane (DODMA).

11. The liposome drug delivery system according to at least one of claims 1 to 10, wherein, the first phospholipid is dioleoylphosphatidylcholine (DOPC), the second phospholipid is distearoylphosphatidylethanolamine-polyethylene glycol 2000 (DSPE-PEG2000), and the mass ratio of the first phospholipid to the second phospholipid is (4-6):(0.4-0.6); the mass ratio of the first phospholipid to the second phospholipid is preferably (4-6):(0.4-0.5), such as 5:0.4 or 5:0.5; or

the first phospholipid is dierucoylphosphatidylcholine (DEPC), the second phospholipid is distearoylphosphatidylethanolamine-polyethylene glycol 2000 (DSPE-PEG2000), and the mass ratio of the first phospholipid to the second phospholipid is (4-6):(0.4-0.6); the mass ratio of the first phospholipid to the second phospholipid is preferably (4-6):(0.4-0.5), such as 5:0.4 or 5:0.5; or
the first phospholipid is 1-palmitoyl-2-oleoyl-phosphatidylcholine (POPC), the second phospholipid is distearoylphosphatidylethanolamine-polyethylene glycol 2000 (DSPE-PEG2000), and the mass ratio of the first phospholipid to the second phospholipid is (4-6):(0.4-0.6); the mass ratio of the first phospholipid to the second phospholipid is preferably (4-6):(0.4-0.5), such as 5:0.4 or 5:0.5.

12. A method for preparing the liposome drug delivery system according to at least one of claims 1 to 11, wherein, the preparation method comprises the following steps:

I, taking and dissolving the deoxycholic acid, the first phospholipid, the second phospholipid, cholesterol, and an antioxidant according to a ratio in an organic solvent to prepare an organic phase;
II, removing the organic solvent in the organic phase obtained in step I, and adding an aqueous phase to the organic phase for hydration, so as to obtain a crude suspension;
III, homogenizing the crude suspension obtained in step II, so as to obtain a suspension a; and
IV, filtering the suspension a obtained in step III through a 0.45 μm filter membrane or a double-layer filter membrane of (1.0+0.65) μm, and then performing filtration sterilization through a 0.22 μm microporous filter membrane, so as to obtain the liposome, wherein the antioxidant is preferably vitamin E acetate.

13. The preparation method according to claim 12, wherein, the total solid content of the organic phase in step I is 20% (w/v); and/or

the organic solvent in step I is selected from one or more of chloroform, anhydrous ethanol, and anhydrous methanol, preferably a mixed solution of chloroform and anhydrous methanol; wherein in the mixed solution of chloroform and anhydrous methanol, the volume ratio of chloroform to anhydrous ethanol is preferably 1:1-30:1, such as 10:1; and/or
the aqueous phase in step II is selected from one or more of a 0.02-0.04 M disodium succinate buffer solution containing 0.5% NaCl, a 0.05 M disodium succinate buffer solution, a 0.025 M histidine solution, normal saline, and an 8% sucrose solution; more preferably, the aqueous phase is the 0.02-0.04 M disodium succinate buffer solution containing 0.5% NaCl; more preferably, the pH value of the 0.02-0.05 M disodium succinate buffer solution containing 0.5% NaCl is 5.0-7.0, such as 6.0-6.5; and/or

the method for removing the organic solvent in step II is reduced-pressure distillation; the reduced-pressure distillation is preferably performed at 45-60 °C; and/or

the temperature for hydration in step II is 20-60 °C, preferably 50-60 °C or 20-25 °C; and/or

the time for hydration in step II is 60-120 min; and/or

the homogenizing condition in step III is to perform homogenizing for 3-5 times at 8000-12000 psi.

14. A liposomal formulation, wherein, the liposomal formulation comprises the liposome drug delivery system according to any one of claims 1 to 11, wherein the preparation is an injection or a lyophilized preparation.

15. The liposomal formulation according to claim 14, wherein, when the preparation is the lyophilized preparation, the preparation further optionally comprises a lyophilized excipient; and the lyophilized excipient is selected from one or more of sucrose, lactose, trehalose, and mannitol, preferably sucrose.

16. The liposomal formulation according to at least one of claims 14 to 15, wherein, when the liposomal formulation is the lyophilized preparation, the method for preparing the lyophilized preparation comprises the method for preparing a liposome drug delivery system according to at least one of claims 12 to 13, and after homogenizing in step III and before filtration using a filter membrane in step IV, the suspension a is mixed with the lyophilized excipient, and then an aqueous phase is added; then filtration is performed through a 0.45 $\mu$m filter membrane, then filtration is performed through a 0.22 $\mu$m microporous filter membrane, so as to obtain a liposome, and the liposome is lyophilized;

the aqueous phase is preferably a 0.025 M disodium succinate buffer solution containing 0.5% NaCl at pH 6-6.5; and

the adding of the aqueous phase preferably causes a weight percentage content of the lyophilized excipient in the solution to reach 5%-8%, preferably 6%.

17. The liposome drug delivery system according to at least one of claims 1 to 11 or the liposomal formulation according to at least one of claims 14 to 16, wherein, the average particle size of the liposome is 50-300 nm, preferably 50-100 nm; more preferably, the average particle size of the liposome is 60-95 nm, and most preferably 60-91.5 nm; and/or

in the liposome drug delivery system or liposomal formulation, Encapsulation Efficiency (EE) of the liposome is above 75%, preferably above 85%, more preferably above 90%, and further preferably above 95%; and/or

in the liposome drug delivery system or liposomal formulation, the Polydispersity Index (PDI) of the liposome is 0.1-0.4, preferably 0.1-0.35, more preferably 0.1-0.25, and most preferably 0.1-0.2; and/or

in the liposome drug delivery system or liposomal formulation, the Zeta potential of the liposome reaches -65 mV to -5 mV, preferably -45mV to -20 mV, and more preferably -45 mV to -35 mV.

18. Use of the liposome drug delivery system according to at least one of claims 1 to 11 or the liposomal formulation according to at least one of claims 14 to 16 in the preparation of a fat-reducing drug or a medical aesthetic product, wherein preferably, the fat-reducing drug or medical aesthetic product is a localized fat-reducing drug or a localized medical aesthetic product.

19. A fat-reducing drug or medical aesthetic product, wherein, the fat-reducing drug or medical aesthetic product comprises the liposome drug delivery system according to at least one of claims 1 to 11 or the liposomal formulation according to at least one of claims 14 to 16, as well as an acceptable excipient or auxiliary component in the fat-reducing drug or medical aesthetic product; and

preferably, the excipient or auxiliary component is selected from one or more of ascorbic acid (VC), benzoic acid, and lidocaine.

20. The fat-reducing drug or medical aesthetic product according to claim 19, wherein, the fat-reducing drug or medical aesthetic product is a liposomal formulation for local injection into adipose tissue.

Fig. 1

Fig. 2

**a** Adipocyte-24h

**b** HUVEC-24h

Fig. 3

## INTERNATIONAL SEARCH REPORT

| International application No. |
|---|
| **PCT/CN2024/120596** |

| | |
|---|---|
| **A. CLASSIFICATION OF SUBJECT MATTER** | |

A61K9/127(2025.01)i; A61K47/24(2006.01)i; A61K47/28(2006.01)i; A61K31/575(2006.01)i; A61P3/04(2006.01)i; A61K8/14(2006.01)i; A61K8/55(2006.01)i; A61K8/63(2006.01)i; A61Q19/06(2006.01)i

According to International Patent Classification (IPC) or to both national classification and IPC

**B. FIELDS SEARCHED**

Minimum documentation searched (classification system followed by classification symbols)

IPC: A61K; A61P; A61Q

Documentation searched other than minimum documentation to the extent that such documents are included in the fields searched

Electronic data base consulted during the international search (name of data base and, where practicable, search terms used)

CNABS, CNTXT, DWPI, VEN, USTXT, WOTXT, EPTXT, STN, CNKI: 四川汇宇制药股份有限公司, 四川汇宇悦迎医药科技有限公司, 张向飞, 徐上虎, 孟国栋, 李瑞如, 张兴旺, 戴天, 杨娅, 李玉皇, 李好雨, 丁兆, 脂质体, 脱氧胆酸, 去氧胆酸, 胆汁酸, 胆酸, 二油酰基磷脂酰胆碱, 减脂, 溶脂, 脂肪细胞, 非脂肪, 选择性, 靶向, liposom+, deoxycholic, bile acid, DOPC, DSPE-PEG2000, adipocyte, HUVEC, target, selecitv+, 302-95-4, 83-44-3

**C. DOCUMENTS CONSIDERED TO BE RELEVANT**

| Category* | Citation of document, with indication, where appropriate, of the relevant passages | Relevant to claim No. |
|---|---|---|
| PX | CN 116889548 A (SICHUAN HUIYU YUEYING PHARMACEUTICAL TECHNOLOGY CO., LTD. et al.) 17 October 2023 (2023-10-17) description, paragraphs [0005] and [0092], and embodiment 7 | 1-20 |
| PX | CN 118258845 A (SICHUAN HUIYU PHARMACEUTICAL CO., LTD. et al.) 28 June 2024 (2024-06-28) claim 10 | 1-20 |
| X | CN 1870977 A (SANOFI-AVENTIS DEUTSCHLAND GMBH) 29 November 2006 (2006-11-29) description, page 1, line 19 to page 2, line 15 and page 5, lines 2-20, and embodiment 1 | 1-20 |
| X | US 2006222695 A1 (ZADINI, Filiberto et al.) 05 October 2006 (2006-10-05) description, paragraphs [0007]-[0011] | 1-20 |
| X | RU 2010147051 A (ZHEBROVSKAYA, F. I. et al.) 27 May 2012 (2012-05-27) claims 3-4 | 1-17 |

☑ Further documents are listed in the continuation of Box C.　　☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 January 2025** | **09 January 2025** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/120596** |

### C. DOCUMENTS CONSIDERED TO BE RELEVANT

| Category* | Citation of document, with indication, where appropriate, of the relevant passages | Relevant to claim No. |
|---|---|---|
| A | CN 116211804 A (BEIJING HUICHENG YIJIAN PHARMACEUTICAL TECHNOLOGY CO., LTD.) 06 June 2023 (2023-06-06)<br>claims 1-3 | 1-20 |
| A | CN 114712514 A (ZHEJIANG UNIVERSITY) 08 July 2022 (2022-07-08)<br>embodiment 1 | 1-20 |
| A | 曹金娜等 (CAO, Jinna et al.). "胆盐脂质体的制备及理化性质研究 (Preparation of Liposomes Incorporating Sodium Deoxycholate and Their Physicochemical Properties)"<br>中国药学杂志 (Chinese Pharmaceutical Journal),<br>Vol. 44, No. 2, 31 January 2009 (2009-01-31),<br>pages 120-123 | 1-20 |

Form PCT/ISA/210 (second sheet) (July 2022)

## INTERNATIONAL SEARCH REPORT
### Information on patent family members

International application No.

**PCT/CN2024/120596**

| Patent document cited in search report | | | Publication date (day/month/year) | Patent family member(s) | | | Publication date (day/month/year) |
|---|---|---|---|---|---|---|---|
| CN | 116889548 | A | 17 October 2023 | None | | | |
| CN | 118258845 | A | 28 June 2024 | None | | | |
| CN | 1870977 | A | 29 November 2006 | DE | 502004008793 | D1 | 12 February 2009 |
| | | | | EP | 1684722 | A2 | 02 August 2006 |
| | | | | EP | 1684722 | B1 | 31 December 2008 |
| | | | | IL | 174525 | A0 | 01 August 2006 |
| | | | | KR | 20060117914 | A | 17 November 2006 |
| | | | | JP | 2007509085 | A | 12 April 2007 |
| | | | | WO | 2005041919 | A2 | 12 May 2005 |
| | | | | WO | 2005041919 | A3 | 23 June 2005 |
| | | | | BRPI | 0415756 | A | 19 December 2006 |
| | | | | DE | 10349979 | A1 | 16 June 2005 |
| | | | | DE | 10349979 | B4 | 18 May 2006 |
| | | | | AU | 2004285264 | A1 | 12 May 2005 |
| | | | | HK | 1096857 | A1 | 15 June 2007 |
| | | | | ES | 2319997 | T3 | 18 May 2009 |
| | | | | ATE | 418965 | T1 | 15 January 2009 |
| | | | | MXPA | 06004081 | A | 27 June 2006 |
| | | | | CA | 2543187 | A1 | 12 May 2005 |
| US | 2006222695 | A1 | 05 October 2006 | None | | | |
| RU | 2010147051 | A | 27 May 2012 | None | | | |
| CN | 116211804 | A | 06 June 2023 | None | | | |
| CN | 114712514 | A | 08 July 2022 | None | | | |

Form PCT/ISA/210 (patent family annex) (July 2022)

**REFERENCES CITED IN THE DESCRIPTION**

**Patent documents cited in the description**

- CN 2023112666525
- CN 102970993 A

- CN 1870977 A